# EUROPEAN PATENT SPECIFICATION

(11) **EP 1 906 916 B1**
(45) Date of publication and mention of the grant of the patent: **29.10.2008**
(21) Application number: 06759259.2
(22) Date of filing: 08.05.2006
(51) Int. Cl.: A61K 9/00, A61K 31/165, A61K 31/542, A61K 31/57

(54) **OPHTHALMIC SUSPENSION COMPRISING AN OPHTHALMIC DRUG, A POLOXAMINE AND A GLYCOL TONICITY-ADJUSTING AGENT, USE OF SAID COMPOSITION FOR THE MANUFACTURE OF A MEDICAMENT FOR TREATING OPHTHALMIC DISORDERS**
OPHTHALMISCHE SUSPENSION AUS EINEM OPHTHALMISCHEN ARZNEIMITTEL, EINEM POLOXAMIN UND GLYKOL-TONIZITÄTSANPASSENDEN MITTEL, VERWENDUNG DER ZUSAMMENSETZUNG ZUR HERSTELLUNG EINES MEDIKAMENTS ZUR BEHANDLUNG VON AUGENERKRANKUNGEN
SUSPENSION OPHTALMIQUE CONTENANT UN PRINCIPE ACTIF OPHTHALMIQUE, DE LA POLOXAMINE ET UN AGENT POUR L'ADAPTATION DE LA TONICITE DE TYPE GLYCOL, EMPLOI DE CETTE COMPOSITION POUR LA PRODUCTION D'UN MEDICAMENT POUR LE TRAITEMENT DES MALADIES OPHTHALMIQUES

(30) Priority: 10.05.2005 US 679510 P
(43) Date of publication of application: 09.04.2008
(73) Proprietor: ALCON INC., CH-6331 Hunenberg (CH)
(72) Inventor: OWEN, Geoffrey, Robert, Southlake, TX 76092-2871 (US); BROOKS, Amy, C., Burleson, TX 76028 (US); GRAFF, Gustav, Cleburne, TX 76031 (US)
(74) Representative: Teipel, Stephan
(86) International application number: PCT/US2006/017607
(87) International publication number: WO 2006/121964

(56) References cited:
- WO-A-02/48300
- US-A1- 2002 107 238
- US-A1- 2004 115 270

## Description

### BACKGROUND OF THE INVENTION

This invention relates to pharmaceutical compositions for treating ophthalmic disorders. In particular, the present invention relates to topically administrable suspension formulations of nepafenac and other ophthalmic drugs.

Nepafenac is also known as 2-amino-3-benzoylphenylacetamide. The topical use of nepafenac and other amide and ester derivatives of 3-benzoylphenylacetic acid to treat ophthalmic inflammation and pain is disclosed in U.S. Patent No. 5,475,034. According to the '034 patent, compositions containing the 3-benzoylphenylacetic acid derivatives can be formulated into a variety of topically administrable ophthalmic compositions, such as solutions, suspensions, gels, or ointments. The compositions optionally contain preservatives, such as benzalkonium chloride, and thickening agents; such as carbomers, hydroxyethylcellulose or polyvinyl alcohol. The '034 patent, however, does not disclose any formulations of nepafenac or other ophthalmic drugs containing a combination of a poloxamine surfactant and propylene glycol.

Attempts have been made to increase the corneal flux of topically administrable drugs for some time. Many glycols, including propylene glycol, are known "penetration enhancers." See, for example, U.S. Patent No. 6,765,001. This patent discloses formulations of corticosteroids for topical application to the skin. The reference formulations contain propylene glycol as a skin penetration enhancer.

Corneal penetration enhancers for topically administrable ophthalmic drugs have also been sought. See, for example, U.S. Patent No. 5,369,095, which discloses the use of dodecyl maltoside as a corneal penetration enhancer. See also, U.S Patent Nos. 6,630,135 and 6,835,392, which in addition to dodecyl maltoside disclose other penetration enhancers for mucosal tissues. These penetration enhancers are intended to increase the corneal penetration of the topically administered drug.

Poloxamer and poloxamine surfactants are known. They are used in contact lens care solutions and therapeutic ophthalmic compositions including anti-inflammatory compositions. See, for example, U.S. Patent Nos. 6,037,328; 6,544,953; 6,486,215; and 5,631,005.

US 2002/107238 describes a gellable aqueous ocular composition comprising a preferably antibacterial agent (e.g. dexamethasone, hydrocortisone, prednisolone) and poloxamine (quantity used for gelling agents in general: 0.1-2.5%).

While poloxamine surfactants (including those commercially available as Tetronic^{®} surfactants) and propylene glycol are separately known to be useful in topically administrable ophthalmic compositions, they have not been used in combination with nepafenac and their combined effect on the corneal penetration of sparingly water-soluble ophthalmic drugs has not been disclosed.

### SUMMARY OF THE INVENTION

The compositions of the present invention are aqueous suspension compositions of nepafenac or other ophthalmic drugs that are sparingly soluble in water. The compositions of the present invention comprise a combination of a poloxamine surfactant and a glycol tonicity-adjusting agent. Unlike conventional suspension compositions, the compositions of the present invention do not contain a water-soluble polymeric suspending or viscosifying agent such as a carbopol.

The present invention is based on the finding that suspension compositions of sparingly-soluble ophthalmic drugs containing a combination of a poloxamine surfactant and a glycol tonicty-adjusting agent show significantly greater corneal penetration than similar compositions that do not contain such a combination of excipients.

### DETAILED DESCRIPTION OF THE INVENTION

Unless indicated otherwise, all ingredient concentrations are presented in units of % weight/volume (% w/v).

As used herein, "sparingly soluble in water" or "sparingly-soluble ophthalmic drug" means a drug that has a solubility limit in water at 25 °C in the range of 0.001 - 0.05 %.

The aqueous compositions of the present invention contain a pharmaceutically effective amount of nepafenac or other sparingly soluble ophthalmic drug. Nepafenac is a known nonsteroidal anti-inflammatory compound. It can be made by known methods. See, for example, U.S. Patent Nos. 5,475,034 and 4,313,949, the entire contents of which are incorporated by reference. The nepafenac compositions of the present invention will generally contain 0.01 - 0.3 % (w/v) nepafenac, preferably 0.03 - 0.1 % (w/v) nepafenac.

Particularly with the enhanced corneal drug flux of the compositions of the present invention, nepafenac can be used to treat ophthalmic disorders not only of the ocular surface but also of the posterior section of the eye. For example, the topically administrable nepafenac compositions of the present invention may be used to treat ocular surface pain, uveitis, scleritis, episcleritis, keratitis, surgically-induced inflammation, endophthalmitis, iritis, atrophic macular degeneration, retinitis pigmentosa, iatrogenic retinopathy, retinal tears and holes, cystoid macular edema, diabetic macular edema, diabetic retinopathy, sickle cell retinopathy, retinal vein and artery occlusion, optic neuropathy, exudative macular degeneration, neovascular glaucoma, corneal neovascularization, cyclitis, sickle cell retinopathy, and pterygium.

The compositions may contain a sparingly soluble drug compound other than nepafenac. For example, the compositions of the present invention may comprise a sparingly soluble carbonic anhydrase inhibitor, such as brinzolamide; an antifungal agent, such as natamycin; a phosphodiesterase IV inhibitor (PDE-IV or PDE-4) inhibitor, such as roflumilast; a receptor tyrosine kinase inhibitor; a steroid, such as fluorometholone, hydrocortisone, dexamethasone, prednisolone, loteprednol, or medrysone; or a nonsteroidal anti-inflammatory agent that is sparingly soluble in water. All of the foregoing are known compounds and can be made by known methods.

In addition to at least one sparingly soluble ophthalmic drug, the compositions of the present invention comprise a poloxamine nonionic surfactant of the formula: 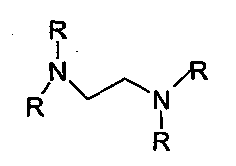 wherein R = 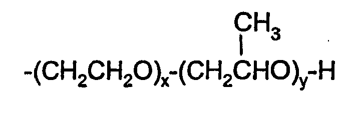 or 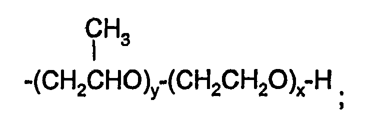 provided that when R is 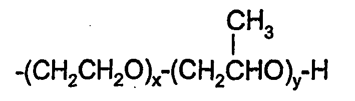 x is 2 -130 and y is 2 - 125, provided that x is 10 - 80% of x + y, and further provided that the number average molecular weight of the poloxamine nonionic surfactant is 1,600 - 30,000,
and when R is 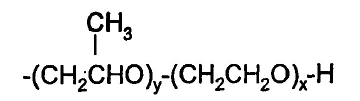 x is 2 - 90 and y is 2 - 90, provided that x is 10 - 80% of x + y, and further provided that the number average molecular weight of the poloxamine nonionic surfactant is 2,600 - 21,000.

Poloxamine nonionic surfactants of the formula above are ethylene diamine initiated poly(oxyethylene) and poly(oxypropylene) block copolymers. They are known and are commercially available as Tetronic^{®} surfactants from BASF Corporation, Performance Products, Florham Park, New Jersey. Poloxamine is the name adopted for such surfactants by The CTFA International Cosmetic Ingredient Dictionary.

The most preferred poloxamine surfactant is that for which R is 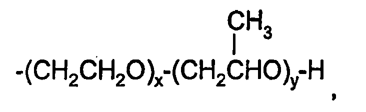 x is about 20, y is about 30, and the number average molecular weight of the poloxamine surfactant is about 10,500. This poloxamine surfactant is commercially available as Tetronic^{®} 1304.

The compositions of the present invention comprise a total of 0.5 - 1.5 % of poloxamine surfactant. Included within the scope of this invention are mixtures of poloxamine surfactants. Higher total concentrations of poloxamine surfactant can reduce the availability of the ophthalmic drug. Preferably, the compositions of the present invention comprise a total of 0.75 - 1.25 % poloxamine surfactant. Most preferably, the compositions of the present invention comprise a total of 1.0 % poloxamine surfactant.

In addition to the ophthalmic drug and the poloxamine surfactant, the compositions of the present invention comprise a glycol tonicity-adjusting agent in a total amount of at least 1 % but less than 4.0 %. The glycol tonicity-adjusting agent is selected from the group consisting of: propylene glycol; glycerol; dipropylene glycol; diethylene glycol; triethylene glycol; 1,3-butylene glycol; 2,3-butylene glycol; 3-methyl-1,3-butylene glycol; diglycerol; erythritol; pentaerythritol; and neopentyl glycol. Included within the scope of this invention are mixtures of glycol tonicity-adjusting agents. Too much glycol tonicity-adjusting agent results in compositions that are uncomfortable when administered because their osmolalities are too high. The compositions of the present invention have osmolalities from 150 - 500 mOsm/Kg. Preferably, the total amount of glycol tonicity-adjusting agent is 2.0 - 3.5 %. Most preferably, the total amount of glycol tonicity-adjusting agent in the compositions of the present invention is 3.0 %. Tonicty-adjusting agents of this type are known and many are commercially available. Preferred glycol tonicity-adjusting agents are propylene glycol, glycerol, and mixtures thereof.

The compositions of the present invention optionally contain metal chloride salts (such as sodium chloride) or non-ionic tonicity adjusting agents (such as mannitol) as additional tonicity-adjusting agents.

The aqueous compositions of the present invention optionally comprise one or more excipients selected from the group consisting of buffering agents, pH-adjusting agents, chelating agents, and preservatives. Buffering agents include phosphate buffers, such as disodium phosphate and monosodium phosphate; borate buffers, such as boric acid and sodium borate; and citrate buffers. The buffering agent is chosen based upon the target pH for the composition, which generally ranges from pH 6.5 - 8.5. The target pH for the composition depends upon the chosen ophthalmic drug. In the case of nepafenac, the desired pH is 7.0 - 8.5, preferably 7.5 - 8.0, and most preferably 7.8. Ophthalmically acceptable pH adjusting agents are known and include, but are not limited to, hydrochloric acid (HCl) and sodium hydroxide (NaOH).

Suitable chelating agents include edetate disodium; edetate trisodium; edetate tetrasodium; and diethyleneamine pentaacetate. Most preferred is edetate disodium. If included, the chelating agent will typically be present in an amount from 0.001 - 0.1 %. In the case of edetate disodium, the chelating agent is preferably present at a concentration of 0.01 %.

Many ophthalmically acceptable preservatives are known and include, but are not limited to, benzalkonium halides and polyquaternium-1. Most preferred preservatives are benzalkonium chloride ("BAC") and polyquaternium-1. In the case of benzalkonium chloride, the preservative is preferably present in an amount from 0.001 - 0.01 %, and most preferably 0.005 %.

The compositions of the present invention optionally comprise a sulfite salt. Examples of sulfite salts include sodium sulfite; potassium sulfite; magnesium sulfite; calcium sulfite; sodium bisulfite; potassium bisulfite; magnesium bisulfite; calcium bisulfite; sodium metabisulfite; potassium metabisulfite; and calcium metabisulfite. If included, the sulfite salt will typically be present in an amount from 0.01 -1 %.

The compositions of the present invention may be prepared by conventional methods of preparing aqueous pharmaceutical suspension compositions, including sizing the drug using known sizing techniques, such as ball-milling. For example, a slurry containing the sparingly soluble drug, a surfactant and sizing beads is tumbled for a time sufficient to obtain drug of desired particle sizes. The sizing beads are then separated from the slurry and the slurry is added to the remaining aqueous ingredients. Preferably, however, the compositions of the present invention are made in a specific manner. According to the preferred method, the drug is first added to a mixture of the poloxamine surfactant and propylene glycol. Preferably, the mixture is warmed (for example, to 50 °C) while the drug is stirred with the mixture to speed up and enhance the dissolution of the drug. After maximizing the dissolution of the drug, the remaining aqueous ingredients (e.g., water, buffering agent, pH-adjusting agent, chelating agent, preservative) are added with vigorous stirring to the dissolved drug. The order of addition to form a mixture of the remaining aqueous ingredients is not critical. This preferred method of preparing the suspension compositions produces a fine suspension of the drug without the need of ball milling to size the drug. In general, target particle sizes for the suspension compositions of the present invention range from 0.1 - 100 µm, and preferably range from 0.5 - 50 µm.

The following examples are intended to illustrate, but not limit, the present invention.

### Example 1

The formulations shown below is representative of the compositions of the present invention.

| | **1** | **1A** |
|---|---|---|
| **INGREDIENT** | % (w/v) | % (w/v) |
| Nepafenac | 0.1 | 0.1 |
| Poloxamine (Tetronic^{®} 1304) | 1.0 | 1.0 |
| Propylene Glycol | 3.0 | 3.0 |
| Edetate Disodium | 0.01 | 0.01 |
| Benzalkonium Chloride | 0.005 | 0.005 |
| Boric Acid | 0.06 | 0.06 |
| Sodium Borate | 0.02 | 0.02 |
| Sodium Sulfite | --- | 0.09 |
| NaOH/HCl | q.s. pH 7.5 - 8.0 | q.s. pH 7.5 - 8.0 |
| Purified Water | q.s. 100 | q.s. 100 |

### Example 2

The formulation shown below is representative of the compositions of the present invention.

| | **2** |
|---|---|
| **INGREDIENT** | % (w/v) |
| PDE-IV Inhibitor | 1.0 |
| Poloxamine (Tetronic^{®} 1304) | 1.0 |
| Propylene Glycol | 3.0 |
| Edetate Disodium | 0.01 |
| Benzalkonium Chloride | 0.005 |
| Disodium Phosphate | 0.1 - 0.2 |
| NaOH/HCl | q.s. pH 7.2 - 8.0 |
| Purified Water | q.s. 100 |

### Example 3

The formulations shown in Table 1 were prepared and evaluated in an *ex vivo* corneal permeation model. The corneal penetration results are also shown in Table 1. Formulations A - C were prepared by ball-milling nepafenac in a slurry containing tyloxapol and/or polysorbate 80 for approximately 18 hours. Formulation D was prepared by dissolving the nepafenac in a mixture of Tetronic^{®} 1304 and propylene glycol, then adding the remaining ingredients. The *ex vivo* corneal penetration rabbit model is briefly described below:
Rabbits were sacrificed by first anaesthetizing with ketamine (30mg/Kg) and xylazine (6mg/Kg) followed by an injection of an overdose of SLEEPAWAY® (sodium pentobarbital, 1ml of a 26% solution) into the marginal ear vein. The intact eyes, along with the lids and conjunctival sacs were then enucleated and immediately stored in about 70 ml of fresh BSS PLUS® irrigation solution saturated with O₂/CO₂ (95:5). Within one hour, the enucleated rabbit eyes were mounted in the modified perfusion chamber as described by Schoenwald, et al., "Corneal Penetration Behavior of β-Blocking Agents I: Physiochemical Factors," Journal of Pharmaceutical Sciences, 72(11) ( November 1983). After mounting in the chambers, 7.5 mls of BSS PLUS® was placed in the receiving side of the chamber with stirring and bubbling and immediately capped to prevent contamination. Then, 7 mls of each test formulation was dosed on the donor side of the chamber for 5 minutes with stirring and bubbling. Afterwards, the donor chamber was emptied with suction and filled with 7mls of BSS PLUS® for approximately 15 seconds. This suction and rinsing with BSS PLUS® was repeated 7 times, and on the 8th fill, the BSS PLUS® was left in the donor chamber. Samples were withdrawn from the receiving chamber every 30 minutes over a five hour period, and the levels of test drug were determined using HPLC. The rate of drug accumulation in the receiver compartment and 5 hour accumulations were then calculated from graphs of the data.

The solubility of the test drug was determined using HPLC analysis after filtering the test formulation through a 0.25 micron screen.

**TABLE 1**

| | Formulation (% w/v) | | | | | |
|---|---|---|---|---|---|---|
| **Ingredient** | **A** | **B** | **C** | **D** | **E** | **F** |
| Nepafenac | 0.1 | 0.1 | 0.1 | 0.1 | 0.1 | 0.1 |
| Carbopol 974P | 0.5 | 0.5 | -- | -- | 0.5 | --- |
| Sodium Chloride | 0.4 | 0.4 | 0.28 | -- | -- | -- |
| Mannitol | 2.4 | 2.4 | -- | -- | -- | -- |
| Tyloxapol | 0.01 | 0.01 | -- | -- | -- | -- |
| Disodium Phosphate | -- | -- | 0.18 | 0.16 | 0.16 | 0.16 |
| Monosodium Phosphate | -- | -- | -- | 0.04 | 0.04 | -- |
| Tetronic® 1304 | -- | -- | -- | 2 | 2 | 3 |
| Propylene Glycol | -- | -- | -- | 2 | 2 | 1 |
| Polyethylene Glycol | -- | -- | 5 | -- | -- | -- |
| Polysorbate 80 | -- | -- | 0.5 | -- | -- | -- |
| Hydroxypropylmethyl cellulose (HPMC 2910) | -- | -- | 0.5 | -- | -- | -- |
| Dodecyl Maltoside | --- | 0.05 | -- | -- | -- | -- |
| Edetate Disodium | 0.01 | 0.01 | --- | 0.01 | 0.01 | 0.01 |
| Benzalkonium Chloride | 0.005 | 0.005 | --- | 0.005 | 0.005 | 0.005 |
| NaOH/HCl q.s. to pH | 7.5 | 7.5 | 7.5 | 7.4 | 7.4 | 7.8 |
| Osmolality (mOsm) | --- | 296 | 330 | 278 | 365 | 187 |
| Solubility (ppm) | 26 | 16 | 49 | 44 | 45 | 66 |
| **Ex Vivo Corneal Penetration Results** | | | | | | |
| Rate of Accumulation (µg/min) | 0.0126 | 0.011 | 0.0108 | 0.025 | 0.008 | 0.0257 |
| Standard Deviation | 0.0007 | 0.002 | 0.0001 | 0.002 | 0.001 | 0.004 |
| 5 hour accumulation (µg) | 4.2 | 3.8 | 3.5 | 6.8 | 2.8 | 7.0 |
| Standard Deviation | 0.2 | 0.6 | 0.1 | 0.6 | 0.4 | 0.7 |

Formulation B is the same as Formulation A with the known penetration enhancer dodecyl maltoside ("DDM") added. The results show that the penetration of B is slightly inferior to A, showing that DDM is not an effective penetration enhancer in the tested formulation.

Formulation C is a viscous formulation containing polyethylene glycol (5%). The solubility of nepafenac is almost doubled compared to Formulation A, but the penetration results are inferior to A.

Formulation D is a formulation according to the present invention. It contains a combination of a poloxamine surfactant and propylene glycol. The solubility and penetration results are superior to A.

Formulation E is the same as Formulation D with the polymeric suspending/viscosifying agent added. The solubility of nepafenac is effectively the same for both Formulations D and E, but the penetration results for Formulation E are much lower than D. These results show that even though the polymeric suspending/viscosifying agent increases viscosity, it retards penetration across the cornea.

Formulation F is another composition according to the present invention. It contains a combination of a poloxamine surfactant and propylene glycol. Formulation F has increased nepafenac solubility compared to Formulation D, but roughly equivalent corneal penetration results compared to D.

### Example 4

The formulations shown in Table 2 were prepared and evaluated in the *ex vivo* corneal penetration model described above. The corneal penetration results are also shown in Table 2. All Formulations were prepared in the same manner as Formulation D.

**TABLE 2**

| | Formulation (% w/v) | | | | | |
|---|---|---|---|---|---|---|
| **Ingredient** | **G** | **H** | **I** | **J** | **K** | **L** |
| Nepafenac | 0.1 | 0.1 | 0.1 | 0.1 | 0.1 | 0.1 |
| Disodium Phosphate | 0.16 | 0.15 | 0.16 | 0.14 | 0.16 | 0.16 |
| Monosodium Phosphate | --- | 0.01 | --- | 0.02 | --- | --- |
| Tetronic® 1304 | --- | 0.5 | 1 | 1.5 | 2 | 3 |
| Propylene Glycol | 3 | 3 | 3 | 3 | 3 | 3 |
| Edetate Disodium | 0.01 | 0.01 | 0.01 | 0.01 | 0.01 | 0.01 |
| Benzalkonium Chloride | 0.005 | 0.005 | 0.005 | 0.005 | 0.005 | 0.005 |
| NaOH/HCl q.s. to pH | 7.8 | 7.8 | 7.8 | 7.8 | 7.8 | 7.8 |
| Osmolality (mOsm) | 439 | 447 | 463 | 456 | 471 | 488 |
| Solubility (ppm) | 15 | 25 | 33, 28 | 48 | 54 | 79 |
| **Ex Vivo Corneal Penetration Results** | | | | | | |
| Rate of Accumulation (µg/mn) | 0.0347 | 0.0411 | 0.0442 | 0.03845 | 0.0254 | 0.0264 |
| Standard Deviation | 0.004 | 0.006 | 0.003 | 0.0001 | 0.005 | 0.001 |
| 5 hour accumulation (µg) | 9.6 | 10.9 | 12 | 11 | 7.17 | 7.39 |
| Standard Deviation | 1.0 | 1.5 | 0.8 | 0.1 | 1.1 | 0.5 |

Each of the formulations shown in Table 2 contains 3 % propylene glycol. The amount of poloxamine surfactant (Tetronic^{®} 1304) is varied from 0 % (Formulation G) to 3 % (Formulation L). The results show that over this range, the solubility of nepafenac increases from 15 ppm to 79 ppm. The penetration data, however, show that corneal drug penetration increases with increasing poloxamine concentration up to a poloxamine concentration of 1 %, then corneal penetration decreases with increasing poloxamine concentration.

### Example 5

The formulations shown in Table 3 were prepared and evaluated in the *ex vivo* corneal penetration model described above. The corneal penetration results are also shown in Table 3. Formulations M and N were prepared in the same manner as Formulation D.

**TABLE 3**

| | **Formulation (% w/v)** | | | | |
|---|---|---|---|---|---|
| **Ingredient** | **A** | **B** | **I** | **M** | **N** |
| Nepafenac | 0.1 | 0.1 | 0.1 | 0.03 | 0.01 |
| Carbopol 974P | 0.5 | 0.5 | --- | --- | --- |
| Sodium Chloride | 0.4 | 0.4 | --- | --- | --- |
| Mannitol | 2.4 | 2.4 | --- | --- | --- |
| Tyloxapol | 0.01 | 0.01 | --- | --- | --- |
| Disodium Phosphate | --- | --- | 0.16 | 0.14 | 0.14 |
| Monosodium Phosphate | --- | --- | --- | 0.02 | 0.02 |
| Tetronic® 1304 | --- | --- | 1 | 1 | 1 |
| Propylene Glycol | --- | --- | 3 | 3 | 3 |
| Dodecyl Maltoside | --- | 0.05 | --- | --- | --- |
| Edetate Disodium | 0.01 | 0.01 | 0.01 | 0.01 | 0.01 |
| Benzalkonium Chloride | 0.005 | 0.005 | 0.005 | 0.005 | 0.005 |
| NaOH/HCl q.s to pH | 7.5 | 7.5 | 7.8 | 7.8 | 7.8 |
| Osmolality (mOsm) | --- | 296 | 463 | 457 | 450 |
| Solubility (ppm) | 26 | 16 | 33,28 | 53 | 25 |
| **Ex Vivo Corneal Penetration Results** | | | | | |
| Rate of Accumulation (µg/min) | 0.0126 | 0.011 | 0.0442 | 0.0147 | 0.0026 |
| Standard Deviation | 0.0007 | 0.002 | 0.003 | 0.0004 | 0.0003 |
| 5 hour Accumulation (µg) | 4.2 | 3.8 | 12 | 4.6 | 1.1 |
| Standard Deviation | 0.2 | 0.6 | 0.8 | 0.2 | 0.06 |

Formulations I, M, and N are identical, except that the concentration of nepafenac varies from 0.01 - 0.1%. The corneal penetration results shown in Table 3 demonstrate that Formulation I, which has the same amount of nepafenac as Formulation A, has significantly greater corneal penetration results than Formulation A. The results also show that Formulation M, which contains only one-third as much nepafenac as Formulation A, has superior penetration results compared to Formulation A.

### Example 6

The formulations shown in Table 4 were prepared and evaluated in the *ex vivo* corneal penetration model described above. The corneal penetration results are also shown in Table 4. Formulations O, P, Q, and R were prepared in the same manner as Formulation D.

**TABLE 4**

| | **Formulation (% w/v)** | | | | |
|---|---|---|---|---|---|
| **Ingredient** | **O** | **P** | **I** | **Q** | **R** |
| Nepafenac | 0.1 | 0.1 | 0.1 | 0.1 | 0.1 |
| Sodium Chloride | 0.38 | --- | --- | --- | --- |
| Disodium Phosphate | 0.14 | 0.14 | 0.16 | --- | 0.14 |
| Monosodium Phosphate | 0.02 | 0.02 | --- | --- | 0.02 |
| Boric Acid | --- | --- | --- | 0.07 | --- |
| Tetronic® 1304 | 1 | 1 | 1 | 1 | 1 |
| Propylene Glycol | 1 | 2 | 3 | 3 | 4 |
| Edetate Disodium | 0.01 | 0.01 | 0.01 | 0.01 | 0.001 |
| Benzalkonium Chloride | 0.005 | 0.005 | 0.005 | 0.005 | 0.005 |
| NaOH/HCl q.s to pH | 7.8 | 7.8 | 7.8 | 7.8 | 7.8 |
| Osmolality (mOsm) | 295 | 302 | 463 | 438 | 600 |
| Solubility (ppm) | 43 | 40 | 33,28 | 51 | 49 |
| **Ex Vivo Corneal Penetration Results** | | | | | |
| Rate of Accumulation (µg/min) | 0.0344 | 0.0406 | 0.0442 | 0.0425 | 0.0454 |
| Standard Deviation | 0.005 | 0.003 | 0.003 | 0.002 | 0.01 |
| 5 hour Accumulation (µg) | 9.8 | 11.6 | 12 | 11.8 | 12.5 |
| Standard Deviation | 1.3 | 0.8 | 0.8 | 0.6 | 2.8 |

Formulations O, P, I, Q, and R are similar. They each contain 0.1 % nepafenac and 1 % poloxamine surfactant, but varying concentrations of propylene glycol from 1 - 4 %. Formulations I and Q contain different buffers, but are otherwise identical. The corneal penetration results for Formulations I and Q are roughly equivalent. The corneal penetration results shown in Table 4 demonstrate that the corneal permeation of nepafenac generally increases with increasing propylene glycol concentration. Increased propylene glycol concentration, however, gives the composition increased osmolality. For topically administrable ophthalmic compositions, the osmolality should be less than 600 mOsm in order that the composition is comfortable upon instillation in the eye.

### Example 7

The formulations shown in Table 5 were prepared and evaluated in the *ex vivo* corneal penetration model described above. The corneal penetration results are also shown in Table 5. Formulation S was prepared in the same manner as Formulation A. Formulation T was prepared in the same manner as Formulation D.

**TABLE 5**

| | **Formulation (% w/v)** | |
|---|---|---|
| **Ingredient** | **S** | **T** |
| Brinzolamide | 1 | 1 |
| Carbomer 974P | 0.4 | --- |
| Boric Acid | --- | 0.07 |
| Mannitol | 3.3 | --- |
| Tyloxapol | 0.025 | --- |
| Sodium Chloride | 0.25 | --- |
| Tetronic® 1304 | --- | 1 |
| Propylene Glycol | --- | 3 |
| Edetate Disodium | 0.01 | 0.01 |
| Benzalkonium Chloride | 0.01 | 0.005 |
| NaOH/HCl q.s to pH | 7.5 | 7.8 |
| Osmolality (mOsm) | 300 | 434 |
| Solubility (ppm) | 380 | 805 |
| **Ex Vivo Corneal Penetration Results** | | |
| Rate of Accumulation (µg/min) | 0.0182 | 0.0869 |
| Standard Deviation | 0.0055 | 0.0047 |
| 5 hour Accumulation | 6.14 | 22.22 |
| Standard Deviation | 1.9 | 1.27 |

The penetration results shown in Table 5 demonstrate that the compositions of the present invention possess superior corneal penetration when the drug is not nepafenac but is another sparingly soluble ophthalmic drug. In this case, the sparingly soluble ophthalmic drug is the carbonic anhydrase inhibitor known as brinzolamide.

### Example 8

The formulations shown in Table 6 were prepared and evaluated in the *ex vivo* corneal penetration model described above. The corneal penetration results are also shown in Table 6. In this case, the composition was held constant as Formulation Q, but the composition was prepared using three different methods. First, the composition was prepared in the same manner as Formulation A; this is labeled "Method I." Next, the composition was prepared in the same manner as Formulation D; this is labeled "Method II." Lastly, the composition was prepared by dissolving the nepafenac in a mixture of poloxamine surfactant and propylene glycol (while warmed), then the appropriate amount of water and milling beads were added and the slurry was ball-milled for about 18 hours. Once ball-milled the beads were separated from the slurry, the remaining aqueous ingredients were added ("Method III").

**TABLE 6**

| **Formulation** | **Rate of Accumulation (µg/min)** | **5 Hour Accumulation (µg)** |
|---|---|---|
| Q (Method I) | 0.0221 ± 0.01 | 6.84 ± 2.6 |
| Q (Method II) | 0.0425 ± 0.002 | 11.8 ± 0.6 |
| Q (Method III) | 0.0473 ± 0.006 | 13.1 ± 1.6 |

The results shown in Table 6 demonstrate that the preferred method of preparing the suspension compositions of the present invention (Method II) results in a composition with superior corneal penetration.

The invention has been described by reference to certain preferred embodiments; however, it should be understood that it may be embodied in other specific forms or variations thereof without departing from its spirit or essential characteristics. The embodiments described above are therefore considered to be illustrative in all respects and not restrictive, the scope of the invention being indicated by the appended claims rather than by the foregoing description.

## Claims

1. A topically administrable aqueous ophthalmic suspension composition comprising
a) an ophthalmic drug having a solubility in water at 25 °C from 0.001- 0.05 % (w/v);
b) a poloxamine nonionic surfactant in an amount of 0.5 - 1.5 % (w/v);
c) a glycol tonicity-adjusting agent selected from the group consisting of propylene glycol; glycerol; dipropylene glycol; diethylene glycol; triethylene glycol; 1,3-butylene glycol; 2,3-butylene glycol; 3-methyl-1,3-butylene glycol; diglycerol; erythritol; pentaerythritol; and neopentyl glycol, in an amount of at least 1.0 % (w/v) but less than 4.0 % (w/v); and
d) water;
wherein the composition has an osmolality from 150 - 500 mOsm/Kg and wherein the poloxamine nonionic surfactant has the formula 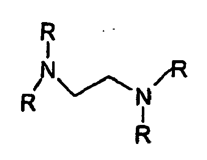
wherein R = 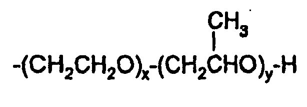 or 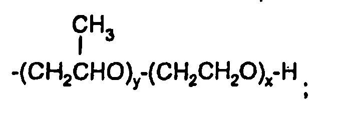 provided that when R is 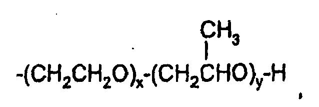 x is 2 - 130 and y is 2 -125, provided that x is 10 - 80% of x + y, and further provided that the number average molecular weight of the poloxamine nonionic surfactant is 1,600 - 30,000,
and when R is 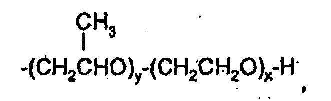 x is 2 - 90 and y is 2 - 90, provided that x is 10 - 80% of x + y, and further provided that the number average molecular weight of the poloxamine nonionic surfactant is 2,600 - 21,000.

2. The composition of Claim 1 wherein the ophthalmic drug is selected from the group consisting of nonsteroidal anti-inflammatory compounds; carbonic anhydrase inhibitors; antifungal agents; phosphodiesterase IV inhibitors; receptor tyrosine kinase inhibitors; and steroids.

3. The composition of Claim 2 wherein the ophthalmic drug is selected from the group consisting of nepafenac; brinzolamide; natamycin; roflumilast; fluorometholone; hydrocortisone; dexamethasone; prednisolone; loteprednol; and medrysone.

4. The composition of Claim 1 wherein the ophthalmic drug is nepafenac.

5. The composition of Claim 1 wherein R is 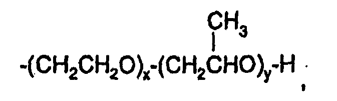 x is about 20, y is about 30, and the number average molecular weight of the poloxamine surfactant is about 10,500.

6. The composition of Claim 1 wherein the poloxamine nonionic surfactant is present in an amount from 0.75 -1.25 % (w/v).

7. The composition of Claim 6 wherein the poloxamine nonionic surfactant is present in an amount of 1.0 % (w/v).

8. The composition of Claim 1 wherein the glycol tonicity-adjusting agent is selected from the group consisting of: propylene glycol; glycerol; and mixtures thereof.

9. The composition of Claim 1 wherein the glycol tonicity-adjusting agent is present in an amount from 2.0 - 3.5 % (w/v).

10. The composition of Claim 9 wherein the glycol tonicity-adjusting agent is selected from the group consisting of: propylene glycol; glycerol; and mixtures thereof.

11. The composition of Claim 10 wherein the glycol tonicity-adjusting agent is present in an amount of 3.0 % (w/v).

12. The composition of Claim 1 wherein the composition further comprises a tonicity-adjusting agent selected from the group consisting of metal chloride salts and non-ionic tonicity adjusting agents.

13. The composition of Claim 1 wherein the composition further comprises an excipient selected from the group consisting of buffering agents; pH-adjusting agents; chelating agents; and preservatives.

14. The composition of Claim 1 wherein the composition lacks a polymeric suspending agent.

15. A topically administrable aqueous ophthalmic suspension composition comprising
a) 0.01 - 0.3 % (w/v) nepafenac;
b) 0.5 -1.5 % (w/v) poloxamine nonionic surfactant;
c) 2.0 - 3.5 % (w/v) glycol tonicity-adjusting agent is selected from the group consisting of: propylene glycol; glycerol; and mixtures thereof;
d) 0.001 - 0.1 % (w/v) edetate disodium;
e) 0.001 - 0.01 % (w/v) of an ophthalmically acceptable preservatives; and
f) water;
wherein the composition has a pH from 7.5 - 8.0 and an osmolality from 250 - 500 mOsm/Kg, and wherein the poloxamine nonionic surfactant has the formula 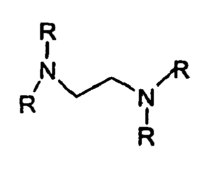
wherein R = 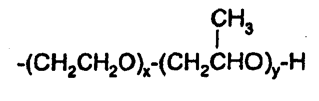 or 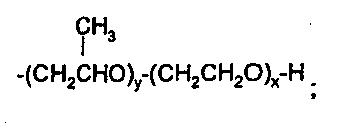 provided that when R is 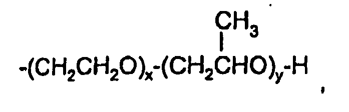 x is 2 - 130 and y is 2 - 125, provided that x is 10 - 80% of x + y, and further provided that the number average molecular weight of the poloxamine nonionic surfactant is 1,600 - 30,000,
and when R is 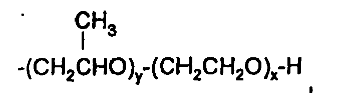 x is 2 - 90 and y is 2 - 90, provided that x is 10 - 80% of x + y, and further provided that the number average molecular weight of the poloxamine nonionic surfactant is 2,600 - 21,000.

16. The composition of Claim 15 wherein the composition further comprises a sulfite salt selected from the group consisting of sodium sulfite; potassium sulfite; magnesium sulfite; calcium sulfite; sodium bisulfite; potassium bisulfite; magnesium bisulfite; calcium bisulfite; sodium metabisulfite; potassium metabisulfite; and calcium metabisulfite.

17. Use of an aqueous suspension composition comprising
a) a pharmaceutically effective amount of nepafenac;
b) a poloxamine nonionic surfactant in an amount of 0.5 - 1.5 % (w/v);
c) a glycol tonicity-adjusting agent in an amount of at least 1.0 % (w/v) but less than 4.0 % (w/v); and
d) water;
wherein the composition has an osmolality from 150 - 500 mOsm/Kg, the poloxamine nonionic surfactant has the formula 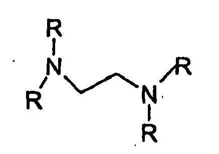
wherein R = 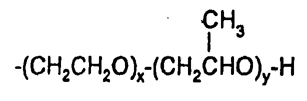 or 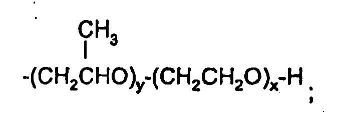 provided that when R is 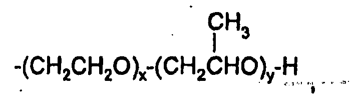 x is 2 -130 and y is 2 - 125, provided that x is 10 - 80% of x + y, and further provided that the number average molecular weight of the poloxamine nonionic surfactant is 1,600 - 30,000,
and when R is 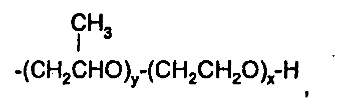 x is 2 - 90 and y is 2 - 90, provided that x is 10 - 80% of x + y, and further provided that the number average molecular weight of the poloxamine nonionic surfactant is 2,600 - 21,000,
the glycol tonicity-adjusting agent is selected from the group consisting of:
propylene glycol; glycerol; dipropylene glycol; diethylene glycol; triethylene glycol; 1,3-butylene glycol; 2,3-butylene glycol; 3-methyl-1,3-butylene glycol; diglycerol; erythritol; pentaerythritol; and neopentyl glycol,
for the manufacture of a topical medicament for the treatment of an ophthalmic disorder, wherein the ophthalmic disorder is selected from the group consisting of ocular surface pain; uveitis; scleritis; episcleritis; keratitis; surgically-induced inflammation; endophthalmitis; iritis; atrophic macular degeneration; retinitis pigmentosa; iatrogenic retinopathy; retinal tears and holes; cystoid macular edema; diabetic macular edema; diabetic retinopathy; sickle cell retinopathy; retinal vein and artery occlusion; optic neuropathy; exudative macular degeneration; neovascular glaucoma; corneal neovascularization; cyclitis; sickle cell retinopathy; and pterygium.

18. The use of Claim 17 wherein the composition comprises
a) 0.01 - 0.3 % (w/v) nepafenac;
b) 0.5 -1.5 % (w/v) of the poloxamine nonionic surfactant;
c) 2.0 - 3.5 % (w/v) of the glycol tonicity-adjusting agent, wherein the glycol tonicity-adjusting agent is selected from the group consisting of: propylene glycol; glycerol; and mixtures thereof;
d) 0.001 - 0.1 % (w/v) edetate disodium;
e) 0.001 - 0.01 % (w/v) of an ophthalmically acceptable preservative; and
f) water;
wherein the composition has a pH from 7.5 - 8.0.

## Patentansprüche

1. Topisch verabreichbare wässerige ophthalmische Suspensionszusammensetzung, umfassend
a) ein ophthalmisches Arzneimittel mit einer Löslichkeit in Wasser bei 25 °C von 0,001 - 0,05 % (Gew./V.);
b) ein nicht-ionisches oberflächenaktives Mittel auf der Basis von Poloxamin in einer Menge von 0,5 - 1,5 % (Gew./V.);
c) ein Tonizitätseinstellmittel auf der Basis von Glycol, ausgewählt aus der Gruppe, bestehend aus Propylenglycol; Glycerol; Dipropylenglycol; Diethylenglycol; Triethylenglycol; 1,3-Butylenglycol; 2,3-Butylenglycol; 3-Methyl-1,3-butylenglycol; Diglycerol; Erythritol; Pentaerythritol und Neopentylglycol, in einer Menge von mindestens 1,0 % (Gew./V.), aber weniger als 4,0 % (Gew./V.); und
d) Wasser;
wobei die Zusammensetzung eine Osmolalität von 150 - 500 mOsm/kg hat und wobei das nicht-ionische oberflächenaktive Mittel auf der Basis von Poloxamin die Formel 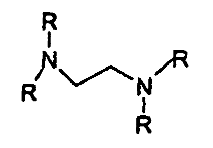 aufweist;
worin R = 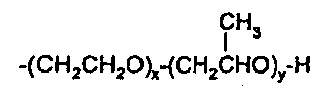 oder 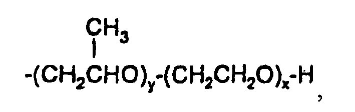 vorausgesetzt, daß, wenn R 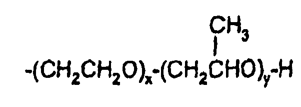 ist,
x 2 - 130 ist und y 2 - 125 ist, vorausgesetzt, daß x 10 - 80 % von x + y ist, und ferner vorausgesetzt, daß das zahlenmittlere Molekulargewicht des nicht-ionischen oberflächenaktiven Mittels auf der Basis von Poloxamin 1.600 - 30.000 beträgt, und wenn R 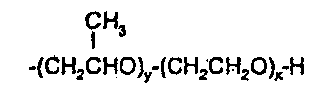 ist,
x 2 - 90 ist und y 2 - 90 ist, vorausgesetzt, daß x 10 - 80 % von x + y ist, und ferner vorausgesetzt, daß das zahlenmittlere Molekulargewicht des nicht-ionischen oberflächenaktiven Mittels auf der Basis von Poloxamin 2.600 - 21.000 beträgt.

2. Zusammensetzung nach Anspruch 1, wobei das ophthalmische Arzneimittel aus der Gruppe ausgewählt ist, bestehend aus nicht-steroidalen entzündungshemmenden Verbindungen; Carboanhydraseinhibitoren; Antimykotika; Phosphodiesterase IV-Inhibitoren; Rezeptortyrosinkinaseinhibitoren und Steroiden.

3. Zusammensetzung nach Anspruch 2, wobei das ophthalmische Arzneimittel aus der Gruppe ausgewählt ist, bestehend aus Nepafenac; Brinzolamid; Natamycin; Roflumilast; Fluorometholon; Hydrocortison; Dexamethason; Prednisolon; Loteprednol und Medryson.

4. Zusammensetzung nach Anspruch 1, wobei das ophthalmische Arzneimittel Nepafenac ist.

5. Zusammensetzung nach Anspruch 1, wobei R 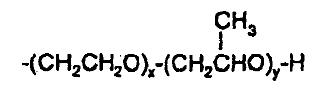 ist,
x etwa 20 ist, y etwa 30 ist und das zahlenmittlere Molekulargewicht des oberflächenaktiven Mittels auf der Basis von Poloxamin etwa 10.500 beträgt.

6. Zusammensetzung nach Anspruch 1, wobei das nicht-ionische oberflächenaktive Mittel auf der Basis von Poloxamin in einer Menge von 0,75 - 1,25 % (Gew./V.) vorliegt.

7. Zusammensetzung nach Anspruch 6, wobei das nicht-ionische oberflächenaktive Mittel auf der Basis von Poloxamin in einer Menge von 1,0 % (Gew./V.) vorliegt.

8. Zusammensetzung nach Anspruch 1, wobei das Tonizitätseinstellmittel auf der Basis von Glycol aus der Gruppe ausgewählt ist, bestehend aus: Propylenglycol; Glycerol und Gemischen davon.

9. Zusammensetzung nach Anspruch 1, wobei das Tonizitätseinstellmittel auf der Basis von Glycol in einer Menge von 2,0 - 3,5 % (Gew./V.) vorliegt.

10. Zusammensetzung nach Anspruch 9, wobei das Tonizitätseinstellmittel auf der Basis von Glycol aus der Gruppe ausgewählt ist, bestehend aus: Propylenglycol; Glycerol und Gemischen davon.

11. Zusammensetzung nach Anspruch 10, wobei das Tonizitätseinstellmittel auf der Basis von Glycol in einer Menge von 3,0 % (Gew./V.) vorliegt.

12. Zusammensetzung nach Anspruch 1, wobei die Zusammensetzung ferner ein Tonizitätseinstellmittel, ausgewählt aus der Gruppe, bestehend aus Metallchloridsalzen und nicht-ionischen Tonizitätseinstellmitteln, umfaßt.

13. Zusammensetzung nach Anspruch 1, wobei die Zusammensetzung ferner einen Hilfsstoff, ausgewählt aus der Gruppe, bestehend aus Puffern, pH-Einstellmitteln, Chelatbildnern und Konservierungsmitteln, umfaßt.

14. Zusammensetzung nach Anspruch 1, wobei die Zusammensetzung kein polymeres Suspendiermittel aufweist.

15. Topisch verabreichbare wässerige ophthalmische Suspensionszusammensetzung, umfassend
a) 0,01 - 0,3 % (Gew./V.) Nepafenac;
b) 0,5 - 1,5 % (Gew./V.) nicht-ionisches oberflächenaktives Mittel auf der Basis von Poloxamin;
c) 2,0 - 3,5 % (Gew./V.) Tonizitätseinstellmittel auf der Basis von Glycol, ausgewählt aus der Gruppe, bestehend aus: Propylenglycol; Glycerol und Gemischen davon;
d) 0,001 - 0,1 % (Gew./V.) Edetatdinatrium;
e) 0,001 - 0,01 % (Gew./V.) eines ophthalmisch akzeptablen Konservierungsmittels und
f) Wasser;
wobei die Zusammensetzung einen pH von 7,5 - 8,0 und eine Osmolalität von 250 - 500 mOsm/kg hat, und wobei das nicht-ionische oberflächenaktive Mittel auf der Basis von Poloxamin die Formel 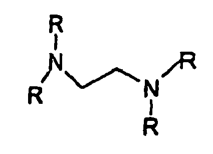 aufweist;
worin R = 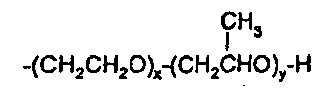 oder 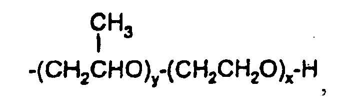 vorausgesetzt, daß, wenn R 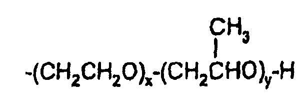 ist,
x 2 - 130 ist und y 2 - 125 ist, vorausgesetzt, daß x 10 - 80 % von x + y ist, und ferner vorausgesetzt, daß das zahlenmittlere Molekulargewicht des nicht-ionischen oberflächenaktiven Mittels auf der Basis von Poloxamin 1.600 - 30.000 beträgt, und wenn R 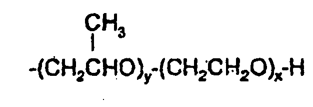 ist,
x 2 - 90 ist und y 2 - 90 ist, vorausgesetzt, daß x 10 - 80 % von x + y ist, und ferner vorausgesetzt, daß das zahlenmittlere Molekulargewicht des nicht-ionischen oberflächenaktiven Mittels auf der Basis von Poloxamin 2.600 - 21.000 beträgt.

16. Zusammensetzung nach Anspruch 15, wobei die Zusammensetzung ferner ein Sulfitsalz, ausgewählt aus der Gruppe, bestehend aus Natriumsulfit; Kaliumsulfit; Magnesiumsulfit; Calciumsulfit; Natriumbisulfit; Kaliumbisulfit; Magnesiumbisulfit; Calciumbisulfit; Natriummetabisulfit; Kaliummetabisulfit und Calciummetabisulfit, umfaßt.

17. Verwendung einer wässerigen Suspensionszusammensetzung, umfassend
a) eine pharmazeutisch wirksame Menge Nepafenac;
b) ein nicht-ionisches oberflächenaktives Mittel auf der Basis von Poloxamin in einer Menge von 0,5 - 1,5 % (Gew./V.);
c) ein Tonizitätseinstellmittel auf der Basis von Glycol in einer Menge von mindestens 1,0 % (Gew./V.), aber weniger als 4,0 % (Gew./V.) und
d) Wasser;
wobei die Zusammensetzung eine Osmolalität von 150 - 500 mOsm/kg hat,
das nicht-ionische oberflächenaktive Mittel auf der Basis von Poloxamin die Formel 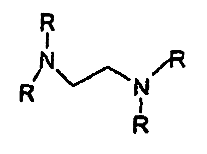 aufweist;
worin R = 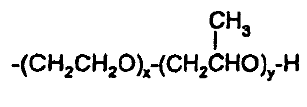 oder 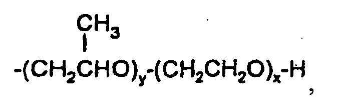 vorausgesetzt, daß, wenn R 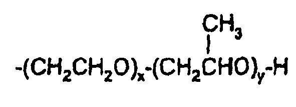 ist,
x 2 - 130 ist und y 2 - 125 ist, vorausgesetzt, daß x 10 - 80 % von x + y ist, und ferner vorausgesetzt, daß das zahlenmittlere Molekulargewicht des nicht-ionischen oberflächenaktiven Mittels auf der Basis von Poloxamin 1.600 - 30.000 beträgt, und wenn R 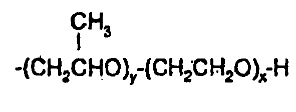 ist,
x 2 - 90 ist und y 2 - 90 ist, vorausgesetzt, daß x 10 - 80 % von x + y ist, und ferner vorausgesetzt, daß das zahlenmittlere Molekulargewicht des nicht-ionischen oberflächenaktiven Mittels auf der Basis von Poloxamin 2.600 - 21.000 beträgt,
das Tonizitätseinstellmittel auf der Basis von Glycol aus der Gruppe ausgewählt ist, bestehend aus: Propylenglycol; Glycerol; Dipropylenglycol; Diethylenglycol; Triethylenglycol; 1,3-Butylenglycol; 2,3-Butylenglycol; 3-Methyl-1,3-butylenglycol; Diglycerol; Erythritol; Pentaerythritol und Neopentylglycol,
zur Herstellung eines topischen Medikaments zur Behandlung einer ophthalmischen Störung,
wobei die ophthalmische Störung aus der Gruppe ausgewählt ist, bestehend aus Augenoberflächenschmerz, Uveitis, Skleritis, Episkleritis, Keratitis, operativ-induzierter Entzündung, Endophthalmitis, Iritis, atropher Makuladegeneration, Retinitis pigmentosa, iatrogener Retinopathie, Netzhautrissen und -löchern, zystoidem Makulaödem, diabetischem Makulaödem, diabetischer Retinopathie, Sichelzellenretinopathie, Netzhautvenen- und -arterienverschluß, optischer Neuropathie, exsudativer Makuladegeneration, neovaskulärem Glaukom, Hornhautneovaskularisation, Ziliarkörperentzündung, Sichelzellenretinopathie und Pterygium.

18. Verwendung nach Anspruch 17, wobei die Zusammensetzung
a) 0,01 - 0,3 % (Gew./V.) Nepafenac;
b) 0,5 - 1,5 % (Gew./V.) des nicht-ionischen oberflächenaktiven Mittels auf der Basis von Poloxamin;
c) 2,0 - 3,5 % (Gew./V.) des Tonizitätseinstellmittels auf der Basis von Glycol,
wobei das Tonizitätseinstellmittel auf der Basis von Glycol aus der Gruppe ausgewählt ist, bestehend aus: Propylenglycol; Glycerol und Gemischen davon;
d) 0,001 - 0,1 % (Gew./V.) Edetatdinatrium;
e) 0,001 - 0,01 % (Gew./V.) eines ophthalmisch akzeptablen Konservierungsmittels und
f) Wasser umfaßt;
wobei die Zusammensetzung einen pH von 7,5 - 8,0 hat.

## Revendications

1. Composition de suspension ophtalmique aqueuse administrable par voie topique, comprenant :
a) un médicament ophtalmique présentant une solubilité dans l'eau à 25 °C de 0,001 à 0,05 % (p/v) ;
b) un tensioactif non ionique de poloxamine en quantité de 0,5 à 1,5 % (p/v) ;
c) un agent isotonisant de type glycol choisi dans le groupe constitué du propylèneglycol ; du glycérol ; du dipropylèneglycol ; du diéthylèneglycol ; du triéthylèneglycol ; du 1,3-butylèneglycol ; du 2,3-butylèneglycol ; du 3-méthyl-1,3-butylèneglycol ; du diglycérol ; de l'érythritol ; du pentaérythritol et du néopentylglycol, en quantité d'au moins 1,0 % (p/v), mais inférieure à 4,0 % (p/v) ; et
d) de l'eau ;
la composition en question ayant une osmolalité de 150 à 500 mOsm/kg et le tensioactif non ionique de poloxamine en question répondant à la formule suivante : 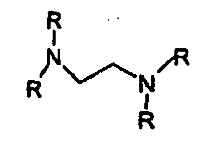
dans laquelle R = 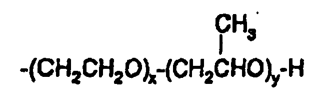 ou 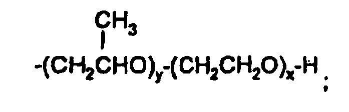 à condition que, lorsque R représente : 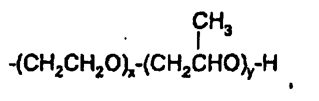 x ait une valeur de 2 à 130 et y ait une valeur de 2 à 125, à condition que x vaille 10 à 80 % de la somme x + y et, en outre, à condition que le poids moléculaire moyen en nombre du tensioactif non ionique de poloxamine se situe de 1600 à 30 000,
et lorsque R représente : 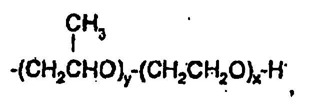 x ait une valeur de 2 à 90 et y ait une valeur de 2 à 90, à condition que x vaille 10 à 80 % de la somme x + y et, également, à condition que le poids moléculaire moyen en nombre du tensioactif non ionique de poloxamine se situe de 2600 à 21 000.

2. Composition selon la revendication 1, dans laquelle le médicament ophtalmique est choisi dans le groupe constitué de composés anti-inflammatoires non stéroïdiens ; d'inhibiteurs de l'anhydrase carbonique ; d'agents antifongiques ; d'inhibiteurs de la phosphodiestérase de type IV ; d'inhibiteurs de récepteurs à activité tyrosine kinase ; et de stéroïdes.

3. Composition selon la revendication 2, dans laquelle le médicament ophtalmique est choisi dans le groupe constitué du népafénac ; du brinzolamide ; de la natamycine ; du roflumilast ; de la fluorométholone ; de l'hydrocortisone ; de la dexaméthasone ; de la prednisolone ; du lotéprednol et de la médrysone.

4. Composition selon la revendication 1, dans laquelle le médicament ophtalmique est le népafénac.

5. Composition selon la revendication 1, dans laquelle R représente le radical de formule suivante : 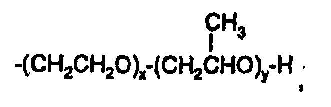 x vaut environ 20, y vaut environ 30 et le poids moléculaire moyen en nombre du tensioactif de poloxamine est d'environ 10 500.

6. Composition selon la revendication 1, dans laquelle le tensioactif non ionique de poloxamine est présent en quantité de 0,75 à 1,25 % (p/v).

7. Composition selon la revendication 6, dans laquelle le tensioactif non ionique de poloxamine est présent en quantité de 1,0 % (p/v).

8. Composition selon la revendication 1, dans laquelle l'agent isotonisant de type glycol est choisi dans le groupe constitué du propylèneglycol ; du glycérol et de leurs mélanges.

9. Composition selon la revendication 1, dans laquelle l'agent isotonisant de type glycol est présent en quantité de 2,0 à 3,5 % (p/v).

10. Composition selon la revendication 9, dans laquelle l'agent isotonisant de type glycol est choisi dans le groupe constitué du propylèneglycol ; du glycérol et de leurs mélanges.

11. Composition selon la revendication 10, dans laquelle l'agent isotonisant de type glycol est présent en quantité de 3,0 % (p/v).

12. Composition selon la revendication 1, dans laquelle la composition comprend, en outre, un agent isotonisant choisi dans le groupe constitué de sels de chlorure de métaux et d'agents isotonisants non ioniques.

13. Composition selon la revendication 1, dans laquelle la composition comprend encore un excipient choisi dans le groupe constitué d'agents à pouvoir tampon ; d'agents d'ajustement du pH ; d'agents chélatants et de conservateurs.

14. Composition selon la revendication 1, dans laquelle un agent de suspension polymère est exempt de la composition.

15. Composition de suspension ophtalmique aqueuse administrable par voie topique, comprenant :
a) 0,01 à 0,3 % (p/v) de népafénac ;
b) 0,5 à 1,5 % (p/v) d'un tensioactif non ionique de poloxamine ;
c) 2,0 à 3,5 % (p/v) d'un agent isotonisant de type glycol, que l'on choisit dans le groupe constitué du propylèneglycol ; du glycérol et de leurs mélanges ;
d) 0,001 à 0,1 % (p/v) d'édétate disodique ;
e) 0,001 à 0,01 % (p/v) d'un conservateur acceptable au plan ophtalmique ; et
f) de l'eau ;
la composition en question ayant un pH de 7,5 à 8,0 et une osmolalité de 250 à 500 mOsm/kg et le tensioactif non ionique de poloxamine en question répondant la formule suivante : 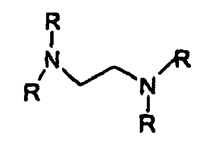
dans laquelle R = 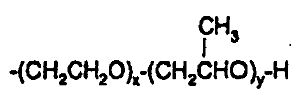 ou 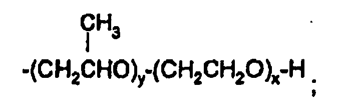 à condition que, lorsque R représente : 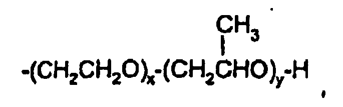 x ait une valeur de 2 à 130 et y une valeur de 2 à 125, pour autant que x vaille 10 à 80 % de la somme x + y et, également, pour autant que le poids moléculaire moyen en nombre du tensioactif non ionique de poloxamine soit de 1600 à 30 000, et lorsque R représente : 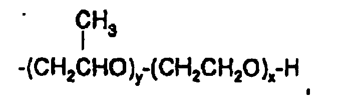 x ait une valeur de 2 à 90 et y une valeur de 2 à 90, pour autant que x vaille 10 à 80 % de la somme x + y et, également, pour autant que le poids moléculaire moyen en nombre du tensioactif non ionique de poloxamine soit de 2600 à 21 000.

16. Composition selon la revendication 15, dans laquelle la composition comprend également un sel de sulfite choisi dans le groupe constitué du sulfite de sodium ; du sulfite de potassium ; du sulfite de magnésium ; du sulfite de calcium ; du bisulfite de sodium ; du bisulfite de potassium ; du bisulfite de magnésium ; du bisulfite de calcium ; du métabisulfite de sodium ; du métabisulfite de potassium et du métabisulfite de calcium.

17. Utilisation d'une composition de suspension aqueuse, comprenant :
a) une quantité de népafénac efficace au plan pharmaceutique ;
b) un tensioactif non ionique de poloxamine en quantité de 0,5 à 1,5 % (p/v) ;
c) un agent isotonisant de type glycol en quantité d'au moins 1,0 % (p/v), mais inférieure à 4,0 % (p/v) ; et
d) de l'eau ;
la composition en question ayant une osmolalité de 150 à 500 mOsm/kg,
le tensioactif non ionique de poloxamine en question répondant à la formule suivante : 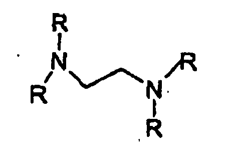
dans laquelle R = 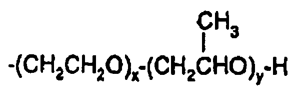 ou 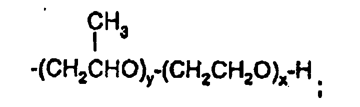 à condition que, lorsque R représente : 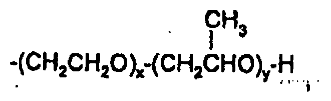 x ait une valeur de 2 à 130 et y une valeur de 2 à 125, à condition que x vaille 10 à 80 % de la somme x + y et, également, à condition que le poids moléculaire moyen en nombre du tensioactif non ionique de poloxamine soit de 1600 à 30 000, et lorsque R représente : 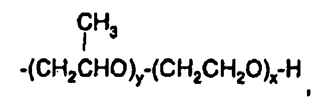 x ait une valeur de 2 à 90 et y une valeur de 2 à 90, à condition que x vaille 10 à 80 % de la somme x + y et, également, à condition que le poids moléculaire moyen en nombre du tensioactif non ionique de poloxamine soit de 2600 à 21 000,
l'agent isotonisant de type glycol étant choisi dans le groupe constitué du propylèneglycol ; du glycérol ; du dipropylèneglycol ; du diéthylèneglycol ; du méthylèneglycol ; du 1,3-butylèneglycol ; du 2,3-butylèneglycol ; du 3-méthyl-1,3-butylèneglycol ; du diglycérol ; de l'érythritol ; du pentaérythritol et du néopentylglycol,
pour la fabrication d'un médicament topique dans le but de traiter un trouble ophtalmique, le trouble ophtalmique étant choisi dans le groupe comprenant une douleur à la surface de l'oeil ; l'uvéite ; la sclérite ; l'épisclérite ; la kératite ; une inflammation induite par voie chirurgicale; l'endophtalmite ; l'iritis ; la dégénération maculaire atrophique ; la rétinite pigmentaire ; la rétinopathie iatrogène, les déchirures et les perforations rétiniennes ; l'oedème maculaire cystoïde ; l'oedème maculaire diabétique ; la rétinopathie diabétique ; la rétinopathie drépanocytaire ; l'occlusion des veines et des artères rétiniennes ; la neuropathie optique ; la dégénération maculaire exsudative ; le glaucome néovasculaire ; la néovascularisation cornéenne ; la cyclite et le ptérygion.

18. Utilisation selon la revendication 17, dans laquelle la composition comprend :
a) 0,01 à 0,3 % (p/v) de népafénac ;
b) 0,5 à 1,5 % (p/v) du tensioactif non ionique de poloxamine ;
c) 2,0 à 3,5 % (p/v) de l'agent isotonisant de type glycol, l'agent isotonisant de type glycol en question étant choisi dans le groupe constitué du propylèneglycol, du glycérol et de leurs mélanges ;
d) 0,001 à 0,1 % (p/v) d'édétate disodique ;
e) 0,001 à 0,01 % (p/v) d'un conservateur acceptable au plan ophtalmique ; et
f) de l'eau ;
la composition ayant un pH de 7,5 à 8,0.
